# EUROPEAN PATENT SPECIFICATION

(11) **EP 1 789 122 B1**
(45) Date of publication and mention of the grant of the patent: **05.06.2019**
(21) Application number: 05770759.8
(22) Date of filing: 12.07.2005
(51) Int. Cl.: A61M 25/16, A61M 25/00, A61F 11/00, A61B 17/34, A61B 17/29

(54) **LAPAROSCOPIC CANNULA ASSEMBLY**
LAPAROSKOPISCHE KANÜLENANORDNUNG
ENSEMBLE AUX CANULES LAPAROSCOPIQUES

(30) Priority: 21.07.2004 US 895546
(43) Date of publication of application: 30.05.2007
(73) Proprietor: Covidien LP, Mansfield, MA 02048 (US)
(72) Inventor: PISKUN, Gregory, Brooklyn, NY 11230 (US); KONIK, Anatoly, 34601 Haifa (IL); ROTTENBERG, Dan, 34601 Haifa (IL)
(74) Representative: Soames, Candida Jane
(86) International application number: PCT/US2005/024636
(87) International publication number: WO 2006/019723

(56) References cited:
- WO-A1-00/18306
- WO-A2-03/001969
- JP-A- 2004 180 858
- US-A- 5 269 772
- US-A- 5 375 588
- US-A- 5 395 327
- US-A- 5 395 367
- US-A- 5 395 367
- US-A- 5 549 637
- US-A- 5 575 799
- US-A- 5 651 771
- US-A1- 2002 049 451
- US-A1- 2003 028 179
- US-A1- 2003 114 832

## Description

### FIELD OF THE INVENTION

The present invention relates to a surgical port assembly. The port assembly is particularly useful in the performance of laparoscopic procedures entirely through the umbilicus.

### BACKGROUND OF THE INVENTION

Abdominal laparoscopic surgery gained popularity in the late 1980's, when benefits of laparoscopic removal of the gallbladder over traditional (open) operation became evident. Reduced postoperative recovery time, markedly decreased post-operative pain and wound infection, and improved cosmetic outcome are well established benefits of laparoscopic surgery, derived mainly from the ability of laparoscopic surgeons to perform an operation utilizing smaller incisions of the body cavity wall.

Laparoscopic procedures generally involve insufflation of the abdominal cavity with C02 gas to a pressure of around 15 mm Hg. The abdominal wall is pierced and a 5-10 mm in diameter straight tubular cannula or trocar sleeve is then inserted into the abdominal cavity. A laparoscopic telescope connected to an operating room monitor is used to visualize the operative field, and is placed through (one of) the trocar sleeve(s). Laparoscopic instruments (graspers, dissectors, scissors, retractors, etc.) are placed through two or more additional trocar sleeves for the manipulations by the surgeon and surgical assistant(s).

Recently, so-called "mini-laparoscopy" has been introduced utilizing 2-3 mm diameter straight trocar sleeves and laparoscopic instruments. When successful, mini- laparoscopy allows further reduction of abdominal wall trauma and improved cosmesis. However, instruments used for mini-laparoscopic procedures are generally more expensive and fragile. Because of their performance limitations, due to their smaller diameter (weak suction-irrigation system, poor durability, decreased video quality), mini-laparoscopic instruments can generally be used only on selected patients with favorable anatomy (thin cavity wall, few adhesions, minimal inflammation, etc.). These patients represent a small percentage of patients requiring laparoscopic procedure. In addition, smaller, 2-3 mm, incisions may still cause undesirable cosmetic outcomes and wound complications (bleeding, infection, pain, keloid formation, etc.).

Since the benefits of smaller and fewer body cavity incisions are proven, it would be attractive to perform an operation utilizing only a single incision in the navel. An umbilicus is the thinnest and least vascularized, and a well-hidden, area of the abdominal wall. The umbilicus is generally a preferred choice of abdominal cavity entry in laparoscopic procedures. An umbilical incision can be easily enlarged (in order to eviscerate a larger specimen) without significantly compromising cosmesis and without increasing the chances of wound complications. The placement of two or more standard (straight) cannulas and laparoscopic instruments in the umbilicus, next to each other, creates a so-called "chopstick" effect, which describes interference between the surgeon's hands, between the surgeon's hands and the instruments, and between the instruments. This interference greatly reduces the surgeon's ability to perform a described procedure.

Thus, there is a need for instruments and trocar systems, which allow laparoscopic procedures to be performed entirely through the umbilicus while at the same time reducing or eliminating the "chopstick effect". A laparoscopic procedure performed entirely through the umbilicus, using the laparoscopic instruments and trocar system according to an embodiment of the present invention, allows one to accomplish the necessary diagnostic and therapeutic tasks while further minimizing abdominal wall trauma and improving cosmesis.

US 2003/0028179 discloses a laparoscopie surgical part on which the preamble of the independent claim is based.

### SUMMARY OF THE INVENTION

According to the invention there is provided a laparoscopic surgical port assembly as set forth in claim 1. The present invention provides instruments and cannula or port assemblies for the performance of surgical procedures, particularly including laparoscopic procedures, for instance, entirely through the umbilicus. The invention aims in part to provide an improved port assembly for facilitating access to internal organs of a patient during laproscopic procedures. The invention also aims to provide such a port assembly that provides enlarged workspace for the hands of the surgeon(s) when plural laparoscopic instruments are placed through the umbilicus. It is within contemplation of the invention to provide improved laparoscopic instruments for facilitating operations through the umbilicus.

The present invention facilitates the performance of laparoscopic surgical procedures wherein several laparoscopic instruments are inserted into a patient through respective cannulas all extending through the same opening in the patient, for instance, through the umbilicus. The advantages of such an operation include minimizing trauma to the patient and accelerating the patient recovery.

A surgical port assembly that facilitates the performance of such a laparoscopic surgical procedure comprises, in accordance with the present invention, a cannula unit including at least one cannula member, and a holder disposable in an opening in a patient's skin for receiving the cannula component so that the cannula component is movable relative to the holder during a surgical procedure. Preferably, the cannula unit is rotatable with respect to the holder about a longitudinal axis of the holder. It is contemplated that the holder is fastened to the patient during the surgical operation, so that the cannula unit is movable relative to the patient.

Pursuant to another feature of the present invention, the cannula unit comprises a base or frame that is removably attachable to the holder and that defines a closure surface extending, during the surgical procedure, substantially tangentially to the patient's skin at the opening. The cannula member is connected to the base and defines an access path through the closure surface. The cannula member extends at an acute angle relative to the closure surface so that the cannula is inclined relative to the patient's skin surface at least in a relaxed (unstressed) or rest configuration of the port assembly, e.g., prior to the insertion of laparoscopic instruments through the cannulas, and preferably during at least a portion of the surgical procedure

Pursuant to further features of the present invention, the cannula is flexible and has a relaxed configuration that is linear. The base or frame is provided with a panel or wall forming the closure surface.

In a particular embodiment of the present invention, the cannula is one of a plurality of cannulas each extending at an acute angle relative to the closure surface so that the cannulas are all inclined relative to the patient's skin surface in a relaxed or rest configuration of the port assembly and during the surgical procedure.

Pursuant to additional features of the present invention, the cannula unit is partially insertable into the holder, while the port assembly further comprises a connector member for removably attaching the cannula unit to the holder, the connector also being partially insertable into the holder.

The holder and the connector member may be provided with cooperating locking elements such as projections and slots for reversibly securing the cannula unit to the holder. Thus, after placement of the holder in an opening in the patient (and after removal of an insert assist member from the holder), the base or frame of the cannula unit is inserted into the holder and secured thereto by an insertion and a rotation of the connector so that the projections and slots are interlocked.

The base or frame of the cannula unit may include a frustoconical portion insertable into the holder. Similarly, the connector may include a frustoconical portion insertable into the holder to secure the cannula unit to the holder. The holder is provided internally with a shoulder engaging a lower end of the cannula unit.

As indicated above, in one embodiment of the present invention, a surgical port assembly comprises (a) a base or frame seatable in an opening in a patient's skin and defining a closure surface extending substantially tangentially to the patient's skin at the opening during a surgical procedure and (b) a cannula connected to the base and defining an access path through the closure surface. The cannula extends at an acute angle relative to the closure surface so that the cannula is inclined relative to the patient's skin surface at least in a relaxed or rest configuration of the port assembly and during the surgical procedure. The cannula may be one of a plurality of cannulas each extending at an acute angle relative to the closure surface so that the cannulas are all inclined relative to the patient's skin surface at least in a relaxed or rest configuration of the port assembly and during the laparoscopic procedure.

Where the closure surface is located in a main plane, the cannulas have linear configurations, and the base or frame has a longitudinal axis, each of the cannulas may define a respective secondary plane oriented perpendicularly to the main plane, each the secondary plane being spaced from the longitudinal axis. The cannulas may be three in number with the secondary planes disposed at angles of 120° relative to each other.

An exemplary surgical method, not forming part of the invention, comprises forming an opening in a patient, inserting a cannula holder through the opening, disposing a plurality of cannulas in the holder so that after inserting of the holder the cannulas traverse the holder and extend from outside the patient to inside the patient, thereafter inserting a plurality of elongate medical instruments through the respective cannulas, and rotating the cannulas and the instruments relative to the holder, about a longitudinal axis of the holder.

In accordance with another aspect of the present invention, where the cannulas are attached to a base member, the disposing of the cannulas in the holder includes inserting the base member into the holder, the base member being in rotatable engagement with the holder. The disposing of the cannulas in the holder may further include attaching a locking member to the holder to maintain the base member in rotatable engagement with the holder.

A laparoscopic medical instrument insertable through a laparoscopic trocar sleeve comprises, in accordance with the present invention, an elongate shaft, an operative tip disposed at one end of the shaft, and a first actuator disposed at an opposite end of the shaft. The actuator is operatively connected to the operative tip via the shaft for controlling the operation of the operative tip. The shaft has a straight proximal end portion, a curved middle portion and a straight distal end portion, the distal end portion extending at an angle with respect to the proximal end portion. The proximal end portion is provided with a first rotary joint so that the distal end portion and the operative tip are rotatable about a longitudinal axis of the proximal end portion. The distal end portion is provided with a second rotary joint so that the operative tip is rotatable about a longitudinal axis of the distal end portion. A second actuator disposed at the end of the shaft opposite the operative tip is operatively connected to the proximal end portion of the instrument shaft for rotating the distal end portion thereof and the operative tip about the longitudinal axis of the proximal end portion of the shaft. A third actuator disposed at the end of the shaft opposite the operative tip is operatively connected to the distal end portion of the shaft for rotating the operative tip about the longitudinal axis of the distal end portion.

The proximal end portion, the middle portion and the distal end portion of the instrument shaft are each substantially rigid throughout so that they cannot be bent. In one embodiment of the invention, the instrument shaft has a hockey-stick shape. Two laparoscopic surgical instruments each having a hockey stick shape are advantageously used in a crossed configuration, which markedly improves the degrees of freedom of the instruments, particularly during lateral (medial-lateral) movements.

In one embodiment of the present invention, a stand-alone laparoscopic medical instrument insertable through a laparoscopic trocar sleeve comprises an elongate shaft, an operative tip disposed at one end of the shaft, and an actuator disposed at an opposite end of the shaft, the actuator being operatively connected to the operative tip via the shaft for controlling the operation of the operative tip. The shaft has a proximal end portion and middle portion and a distal end portion, at least the distal end portion being independently bendable to form a C shape. The distal segments of the shaft are rotatable about a longitudinal axis at least at one location along the instrument's shaft, preferably at the operative tip or proximal to the operative tip.

Pursuant to an additional feature of the present invention, a lock is operatively connected to the shaft for releasably maintaining the C-shaped curved configuration. Moreover, the distal end portion may be provided with an articulated joint, whereby the distal end portion is swingable relative to the middle portion of the shaft. The proximal end portion may also independently bendable to form a C shape.

A stand-alone laparoscopic medical instrument insertable through a laparoscopic trocar sleeve comprises, in accordance with another embodiment of the present invention, an elongate shaft, an operative tip disposed at one end of the shaft, and a manual actuator disposed at an opposite end of the shaft, the manual actuator being operatively coupled to the operative tip via the shaft. A first mechanism is operatively connected to the shaft for bending a proximal portion of the shaft in a first direction, while a second mechanism is operatively connected to the shaft for bending a distal portion of the shaft in a second direction different from the first direction, whereby the shaft assumes a shape with a plurality of differently shaped segments.

The shaft has a longitudinal axis at the one end, and the instrument further comprises a rotation mechanism operatively connected to the shaft for rotating the operative tip about the axis.

Pursuant to another feature of this other embodiment of the invention, first locking element is operatively connected to the first mechanism and a second locking element is operatively connected to the second mechanism, whereby the proximal portion and the distal portion may be maintained as the differently shaped segments. Where the proximal portion and the distal portion are bendable by the first mechanism and the second mechanism in a common plane, the instrument may further comprise an additional mechanism operatively connected to the shaft for bending the distal portion of the shaft in an additional direction out of the plane.

Where the proximal portion of the shaft assumes a first C-shaped configuration in response to operation of the first mechanism and the distal portion of the shaft assumes a second C-shaped configuration in response to operation of the second mechanism, the C-shaped configurations may face opposite sides of the shaft.

A laparoscopic medical instrument comprises, in accordance with an additional embodiment of the present invention, an elongate flexible shaft, an operative tip disposed at one end of the shaft, and a manual actuator disposed at an opposite end of the shaft, the manual actuator being operatively coupled to the operative tip via the shaft, a first bending mechanism being operatively connected to the shaft for curving a proximal portion of the shaft in a first direction, and a second bending mechanism being operatively connected to the shaft for curving a distal portion of the shaft in a second direction different from the first direction, whereby the shaft assumes a shape with a plurality of arcuate segments.

The instrument of this additional embodiment of the invention may additionally comprise a rotation mechanism operatively connected to the shaft for rotating the operative tip about the axis, a first locking element operatively connected to the first bending mechanism and a second locking element operatively connected to the second bending mechanism. The bending mechanisms each include a manual actuator mounted to the shaft at the other end thereof. The invention is limited by the scope of the appended independent claim. Preferred embodiments are disclosed in the dependent claims.

### BRIEF DESCRIPTION OF THE DRAWINGS

Fig. 1 is a perspective view of a laparoscopic port having multiple cannulas, in accordance with the present invention.
Fig. 2 is a cross-sectional view of the laparoscopic port or cannula assembly of Fig. 1, taken along line II-II in Fig. 3.
Fig. 3 is a top view of the laparoscopic port or cannula assembly of Fig. 1.
Fig. 4 is a perspective view of an annular holder disposable in an opening in a patient for receiving the laparoscopic port or cannula assembly of Figs. 1-3.
Fig. 5 is a top plan view of the port holder of Fig. 4.
Fig. 6 is a longitudinal cross-sectional view of the port holder of Figs. 4 and 5, taken along line VI-VI in Fig. 5.
Fig. 7 is another longitudinal cross-sectional view of the port holder of Figs. 4 and 5, taken along line VII-VII in Fig. 5.
Fig. 8 is a perspective view of the laparoscopic port or cannula assembly of Figs. 1-3, together with the port holder of Figs. 4-7, showing the laparoscopic port or cannula assembly inserted into and attached to the port holder.
Fig. 9 is a top plan view of the laparoscopic port or cannula assembly of Figs. 1-3 connected to the port holder of Figs. 4-7, as shown in Fig. 8.
Fig. 10 is a longitudinal cross-sectional view taken along line X-X in Fig. 9.
Fig. 11 is a perspective view of a cannula with an insufflation valve, included in the laparoscopic port or cannula assembly of Figs. 1-3 and 8-10.
Fig. 12 is a side elevational view of the cannula of Fig. 11, on a larger scale.
Fig. 13 is a longitudinal cross-sectional view of the cannula of Figs. 11 and 12, taken along line XIII-XIII in Fig. 12.
Fig. 14 is a transverse cross-sectional view of the cannula of Figs. 11 and 12, taken along line XIV-XIV in Fig. 12.
Fig. 15 is a perspective view of a cannula without an insufflation valve, included in the laparoscopic port or cannula assembly of Figs. 1-3 and 8-10.
Fig. 16 is a side elevational view of the cannula of Fig. 16, on a larger scale.
Fig. 17 is a longitudinal cross-sectional view of the cannula of Figs. 15 and 16, taken along line XVII-XVII in Fig. 16.
Fig. 18 is a transverse cross-sectional view of the cannula of Figs. 15 and 16, taken along line XVIII-XVIII in Fig. 16.
Fig. 19 is a perspective view of an insertion plug used to facilitate insertion of the port holder of Figs. 4-7 in a patient at the beginning of a laparoscopic procedure.
Fig. 20 is a top perspective view of the insertion plug of Fig. 19 temporarily inserted in and attached to the port holder of Figs. 4-7.
Fig. 21 is a top plan view of the assembled insertion plug and port holder of Fig. 20.
Fig. 22 is a longitudinal cross-section taken along line XXII-XXII in Fig. 21.
Fig. 23 is a bottom perspective view of the assembled insertion plug and port holder of Figs. 20-22.
Fig. 24 is a side elevational view of a laparoscopic instrument utilizable with the multiple-cannula port assembly of Figs. 8-10, in accordance with the present invention.
Fig. 25 is a top plan view of the laparoscopic instrument of Fig. 24.
Fig. 26 is partially a side elevational view and partially a cross-sectional view taken along line XXVI-XXVI in Fig. 25.
Fig. 27 is a partial longitudinal cross-sectional view also taken along line XXVI-XXVI in Fig. 25.
Fig. 28 is a side elevational view, on a larger scale, of a distal end of the laparoscopic instrument of Figs. 24-27.
Fig. 29 is a longitudinal cross-sectional view, on an even larger scale, taken along line IXXX-IXXX in Fig.28.
Fig. 30 is a schematic side elevational view of a laparoscopic instrument in accordance with the present invention.
Fig. 31 is a schematic side elevational view of another laparoscopic instrument in accordance with the present invention.
Figs. 32A-32F are diagrams of the instrument of FIG. 31, showing different possible operational configurations of the instrument.
Fig. 33 is a schematic cross-sectional view of a laparoscopic instrument or cannula holder.

### DETAILED DESCRIPTION OF THE PREFERRED EMBODIMENTS

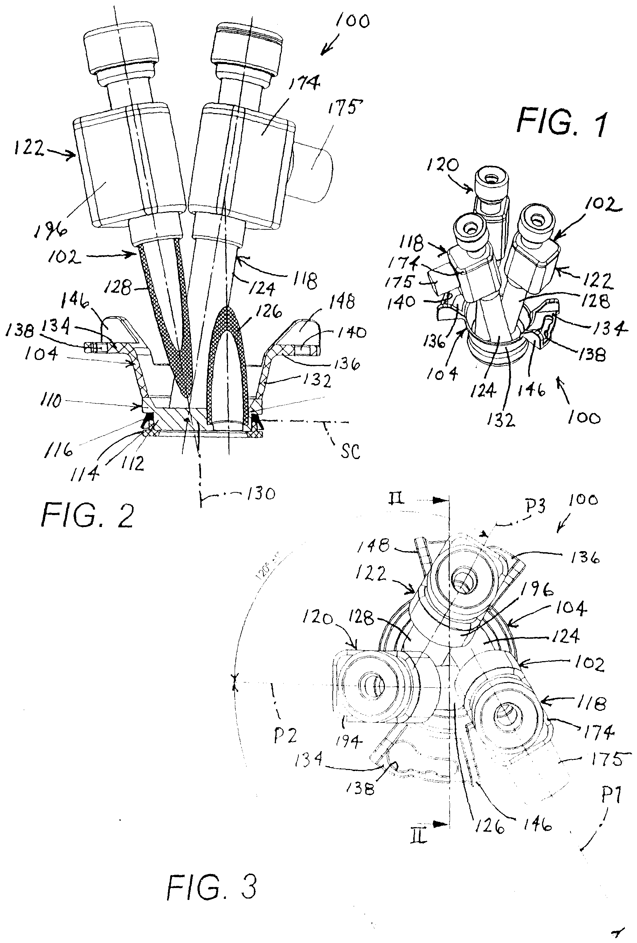

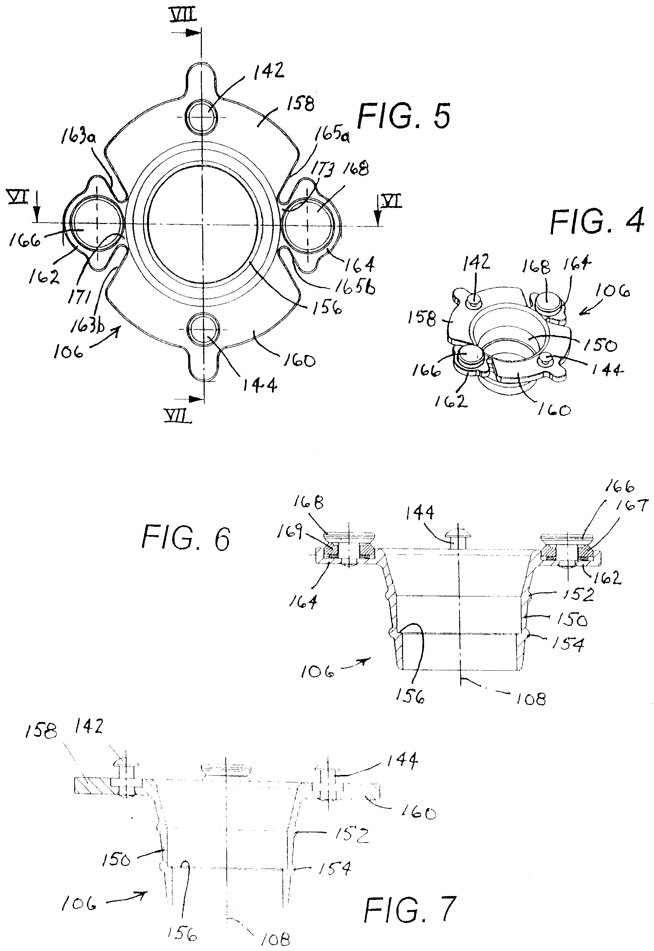

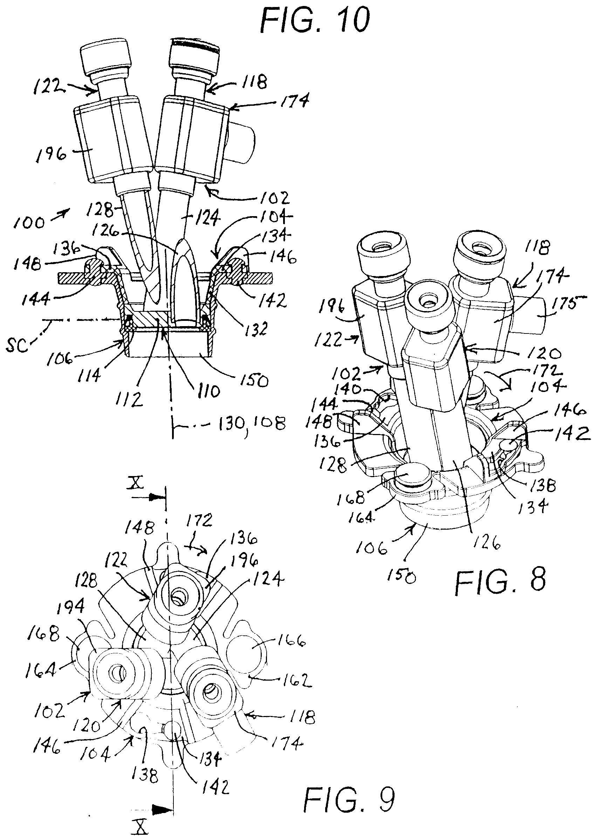

As depicted in Figs. 1-3, a laparoscopic port or cannula assembly 100 comprises a cannula unit 102 and a connector 104 associated therewith for removably fastening the cannula unit to an annular port holder 106 (Figs. 4-8 and 10) that is disposed in an opening (e.g., formed in the umbilicus) in a patient. Cannula unit 102 is coupled to port holder 106 by connector 104 so as to permit rotation of cannula unit 102 about a longitudinal axis 108 (Fig. 7 and 10) of holder 106. Longitudinal axis 108 typically extends approximately perpendicularly to a patient's skin surface when the port assembly 100 has been deployed in that skin surface.

Cannula unit 102 comprises a base or frame 110 that is insertable into and removably attachable to port holder 106. Base or frame 110 includes a planar panel or wall 112 defining a closure surface or plane SC extending, during a laparoscopic surgical procedure, substantially tangentially to the patient's skin at the opening through with port holder 106 extends. Base or frame 110 further includes a seating ring 114 and a sealing ring 116.

Cannula unit 102 additionally comprises three cannula members 118, 120, 122 each connected to base or frame 110 and defining a respective access path through closure surface SC. Cannula members 118, 120, 122 each extend at an acute angle relative to closure surface SC so that the cannulas are inclined relative to the patient's skin surface at least in a relaxed or rest configuration of the port assembly and during a laparoscopic surgical procedure. Cannula members 118, 120, 122 include flexible tubular portions 124, 126, 128 that have linear configurations in a relaxed or unstressed condition.

Each cannula member 118, 120, 122 defines a respective plane P1, P2, P3 (Fig. 3) oriented perpendicularly to closure surface or plane CS (the main plane) and spaced from a longitudinal axis 130 of base or frame 110. These secondary planes P1, P2, and P3 are disposed at angles of 120° relative to each other, as indicated in Fig. 3. Connector 104 is loosely coupled to cannula unit 102 so as to be freely movable along axis 130 of the cannula unit, between base 110 and valve components of cannulas 118, 120, 122.

Connector 104 includes a frustoconical portion 132 insertable into port holder 106 (see Fig. 10) and further includes a pair of flanges 134, 136 for temporarily locking cannula unit 102 to holder 106. To that end, flanges 134, 136 are provided with dual-lobed slots 138, 140 for receiving respective pins or projections 142, 144 on port holder 106 (see Figs. 4, 5, 7, 8, 9). Flanges 134 and 136 are also provided with respective pairs of upturned ears 146 and 148 functioning in part as thumb and finger rests for swiveling connector 104 about axis 108 (and 130) to reversibly secure connector 106 and concomitantly cannula unit 102 to port holder 106.

As illustrated in Figs. 4-7, port holder 106 includes a tapered, slightly frustoconical sleeve 150 provided along an outer surface with a pair of annular beads or ribs 152 and 154 and along an inner surface with a shoulder 156 that serves as an abutment or rest for seating ring 114 of cannula unit 102. At a wider end of sleeve 150, holder 106 includes a pair of diametrically opposed flanges 158, 160 and a pair of diametrically opposed ears 162, 164. Pins or projections 142, 144 are rigid with flanges 158, 160, while ears 162, 164 carry respective flat-headed posts 166, 168 around which sutures are wound to fasten holder 106 to the skin of the patient. Rubber gaskets 167 and 169 may be provided for clamping suture threads to posts 166 and 168. Alternatively or additionally, sutures anchoring port holder 106 to the body wall of the patient may be inserted through slots 163a, 163b and 165a, 165b and partially wrapped around bases 171 and 173 of ears 162, 164 (see Fig. 5).

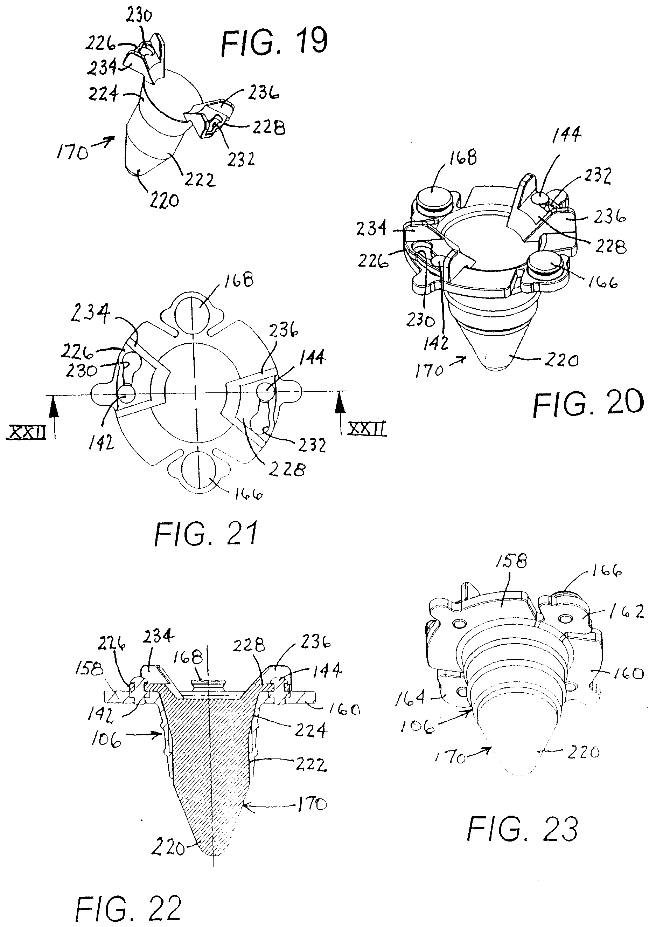

After placement of holder 106 in an opening in the patient (and after removal of an insert assist member 170, Figs. 19-22, from the holder), base or frame 110 of cannula unit 102 is inserted into holder 106 until seating ring 114 engages shoulder 15 (see Fig. 10). Cannula unit 110 is secured to holder 106 by an insertion and a subsequent rotation of connector 104 relative to holder 106, as indicated by an arrow 172 in Figs 8 and 9, so that projections 142, 144 and slots 138, 140 are interlocked (see Figs. 8-10). After this locking of connector 104 to holder 106, cannula unit 102 is rotatable about axes 108 and 130 in opposition to a frictional drag force exerted by virtue of sealing ring 116.

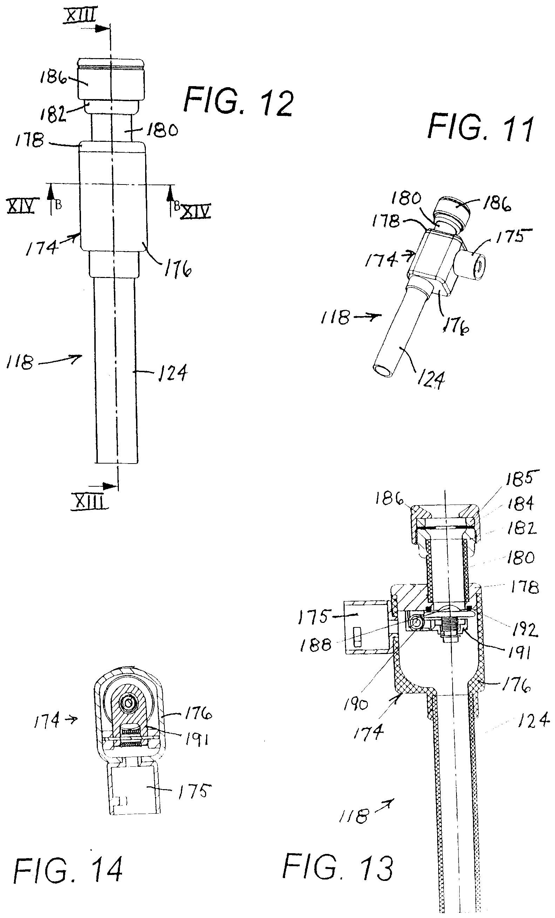

As illustrated in Figs. 11-14, cannula unit 118 includes valve component 174 connected to tubular portion 124. Valve component 124 includes an insufflation port 175 for receiving a tube (not shown) for guiding carbon dioxide gas from a pressurized source into the patient. As shown particularly in Fig. 13, valve component 124 of cannula member 118 includes a valve box or casing 176 with a cover or closure 178 to which an extension tube 180 is attached. At an end opposite valve casing 176, extension tube 180 is provided with a sleeve 182, a valve seal 184, a disc 185, and a cap 186. Valve component 174 further includes a valve door 188 that is biased into a closure position shown in Fig. 13 by a helical or coil spring 190. Door 188 is supported by a mounting bracket and associated hardware 191. An O-ring seal 192 is provided for inhibiting the escape of insufflation gas from a patient through extension tube 124 when a laparoscopic surgical instrument does not traverse cannula member 118.

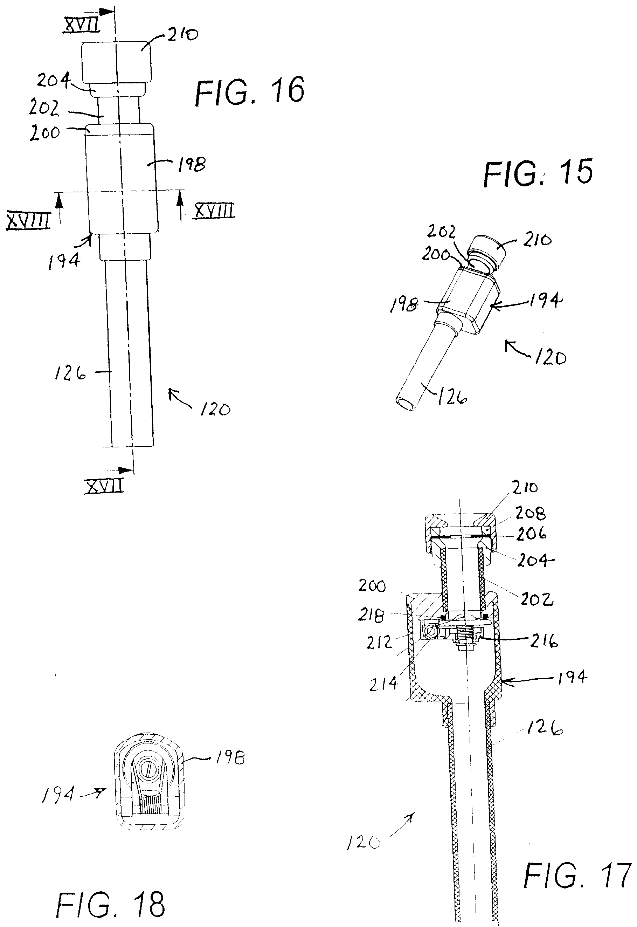

As illustrated in Figs. 1-3 and 8-10, cannula members 120 and 122 include respective valve components 194 and 196 connected to respective flexible tubular portions 126 and 128. These valve components are structurally identical, a representative component 194 being depicted in Figs. 15-18. Valve component 194 includes a valve box or casing 198 with a cover or closure 200 to which an extension tube 202 is attached. At an end opposite valve casing 198, extension tube 202 is provided with a sleeve 204, a valve seal 206, a disc 208, and a cap 210. Valve component 194 further includes a valve door 212 that is biased into a closure position shown in Fig. 17 by a helical or coil spring 214. Door 212 is supported by a mounting bracket and associated hardware 216. An O-ring seal 218 is provided for inhibiting the escape of insufflation gas from a patient through extension tube 202 when a laparoscopic surgical instrument does not traverse the respective cannula member 120 (or 122).

As shown in Fig. 19, insert assist member 170 includes a rounded conical tip 220, a cylindrical middle portion 222 and a slightly tapered or frustoconical outer portion 224. Outer portion 224 is provided with a pair of flanges 226, 228 for temporarily locking insert assist member 170 to port holder 106. To that end, flanges 226, 228 are provided with dual-lobed slots 230, 232 for receiving respective pins or projections 142, 144 on port holder 106, as depicted in Figs. 20-22. Flanges 226 and 228 are formed with respective pairs of upturned ears 234 and 236 that are manually engageable by a user to reversibly secure connector insert assist member 170 to port holder 106.

After a small incision or opening is made in a patient, port holder 106 with insert assist member 170 connected thereto is inserted through the incision. Sutures (not shown) are stitched to the patient and are wound around and tied to posts 166, 168 to firmly secure the port holder 106 to the patient. Insert assist member 170 is then removed, by a reverse rotation unlocking flanges 226, 228 from pins or projections 142, 144 and by separating the inset assist member from holder 106. Cannula unit 102 is then attached to holder 106 as described above.

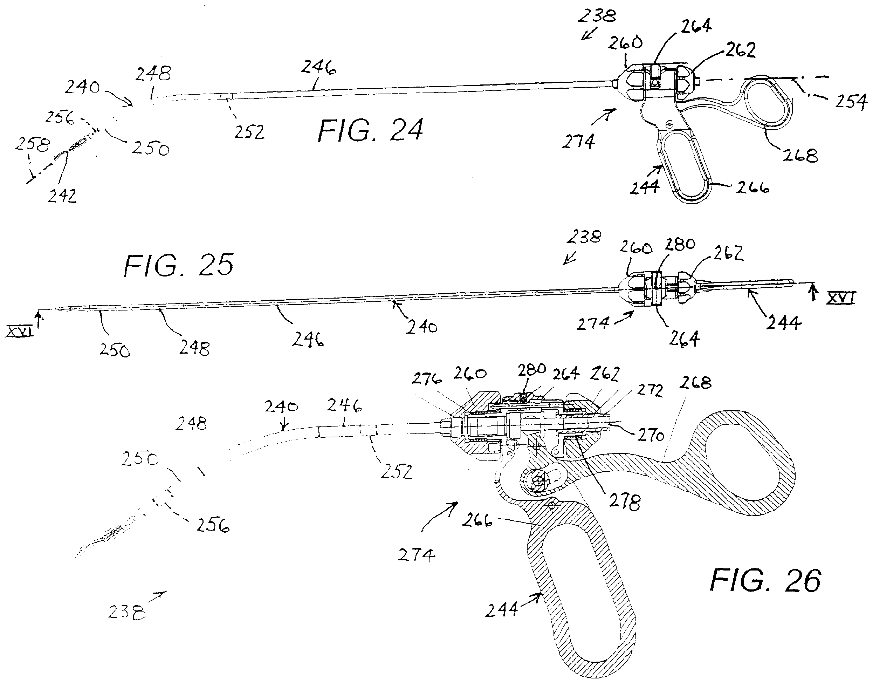

Figs. 24-29 depict a laparoscopic surgical instrument 238 insertable through a laparocopic trocar sleeve or cannula such as cannula member 118, 120, or 122 of the port assembly of Figs. 1-3 and 8-10 for executing a laparoscopic surgical operation. Instrument 238 comprises an elongate shaft 240, an operative tip 242 disposed at one end of the shaft, and a hand-grip-type actuator 244 disposed at an opposite end of the shaft. Actuator 244 is operatively connected to operative tip 242 via shaft 240 for controlling the operation of the operative tip.

Shaft 240 has a straight proximal end portion 246, a curved middle portion 248 and a straight distal end portion 250, the distal end portion extending at a non-zero angle with respect to the proximal end portion, as shown in Figs. 24, 26 and 27. Proximal end portion 246 is provided with a first rotary joint 252 so that distal end portion 250 and operative tip 242 are rotatable about a longitudinal axis 254 of proximal end portion 246. Distal end portion 250 is provided with a second rotary joint 256 so that operative tip 242 is rotatable about a longitudinal axis 258 of the distal end portion. A rotary actuator or knob 260 disposed at the proximal end of instrument 238 is operatively connected to proximal end portion 246 of instrument 240 for rotating distal end portion 250 and operative tip 242 about longitudinal axis 254. Another rotary actuator or knob 262 disposed at the proximal end of instrument 238 is operatively connected to distal end portion 250 of shaft 240 for rotating operative tip 242 about longitudinal axis 258.

Proximal end portion 246, middle portion 248 and distal end portion 250 of instrument shaft 240 are each substantially rigid throughout and can only be rotated about joints 252 and 256 and not bent. The angle between axes 254 and 258 are such that shaft 240 has a shape reminiscent of a hockey stick. In an alternative embodiment of instrument 238, middle portion 248 of shaft 240 may be flexible to permit shaft 240 to alternately assume a linear configuration and the hockey-stick configuration of Figs. 24 and 26. In that case, a handle assembly 274 is provided with an actuator (not shown) for enabling a bending of middle portion 248.

As shown in Figs. 24-26, instrument 238 may be provided with further actuators, such as a slidable toggle switch 264, for example for performing a locking function or inducing a pivoting of operative tip 242 about an axis perpendicular to axis 258.

Actuator 244 includes a hand grip member 266 fixed relative to shaft 240 and further includes a pivotable hand grip 268. A proximal end 270 of shaft 240 is journaled in a bearing 272 about which rotary knob 262. Actuator 244, rotary knobs 260 and 262, and toggle switch 264 are parts of a handle assembly 274 also incorporating yokes 276 and 278, a stopper pin 278, a set screw 280.

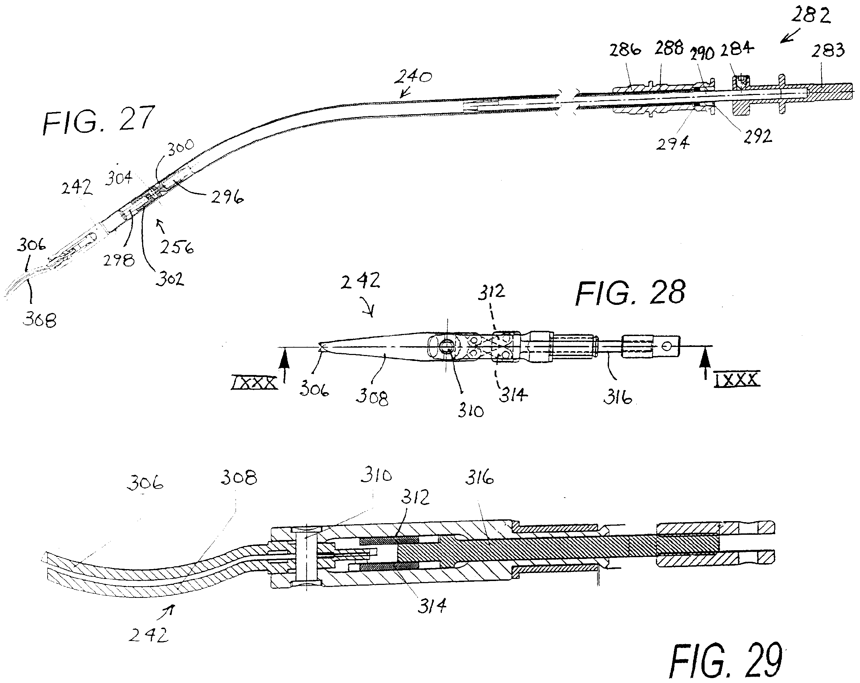

Fig. 27 illustrates further parts of a shaft assembly 282 including shaft 240, a slider member 283, a socket set screw 284, an outer bearing 286, a motion bar 288, a bend tube 290, a ring 292, and an O-ring seal 294.

Rotary joint 256 is representative of joint 254 and comprises, as shown in Fig. 27, a proximal pin or inner shaft portion 296, a distal pin or inner shaft portion 298, a pair of coupling elements 300 and 302, and a transverse connector pin 304.

As illustrated in Figs. 28 and 29, operative tip 242 exemplarily includes a pair of jaws 306 and 308 pivotably connected to a distal end of distal end portion 250 via a pivot pin 310. Jaws 306 and 308 are rotatable about pin 310 through the action of levers or arms 312, 314 that pivot in response to a longitudinal motion of a tip rod 316.

After a deployment of cannula unit 102 in a patient as described above, opeative tip 242 and shaft 240 of instrument 238 are insertable through a cannula member 118, 120, or 122, with the respective tubular portion 124, 126, or 128 bending to accommodate the bent shaft 240. The bent shape of shaft 240, as well as the rotary joints 252 and 256 facilitate the performance of laparoscopic surgical procedure using multiple laparoscopic instruments extending through a single opening in a patient, for instance, in the umbilicus. Such a procedure involves the rotation of distal end portion 250 and operative tip 242 together about axis 254 and the rotation of operative tip about axis 258. In addition, the entire instrument assembly including cannula unit 102 and multiple instruments 238 can be rotated about collinear axes 108 and 130, to optimize the simultaneous or successive access of multiple operative tips 242 to a surgical site inside a patient.

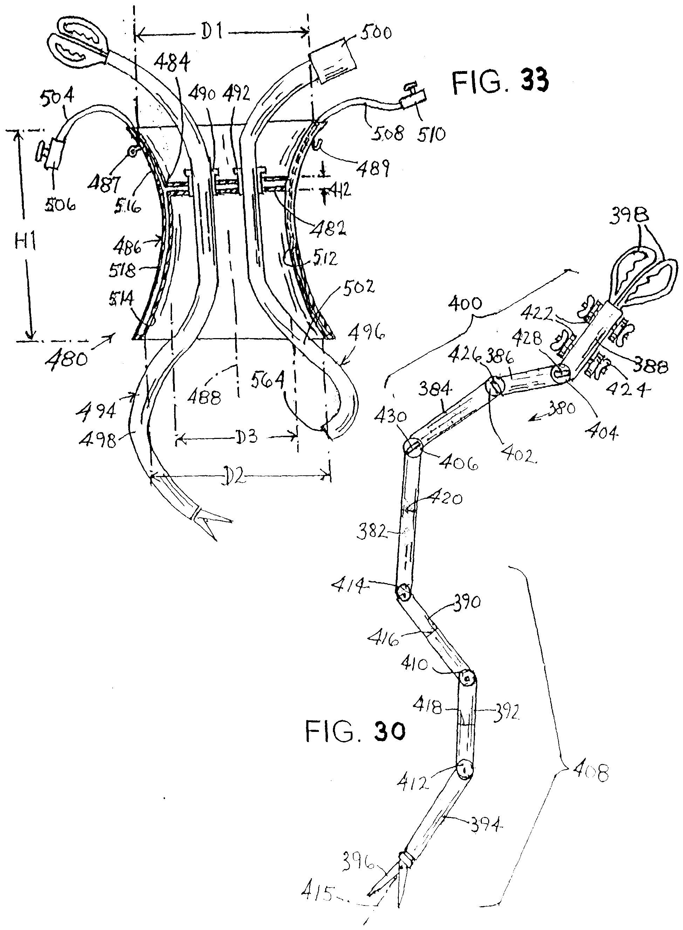

As illustrated in Fig. 30, a stand-alone laparoscopic medical instrument insertable through a laparoscopic trocar sleeve or cannula comprises an elongate shaft 380 formed of a plurality of a plurality of rigid cylindrical segments including a middle segment 382, three proximal end segments 384, 86, and 388, and three distal end segments 390, 392, and 394. During a laparoscopic procedure utilizing the instrument of Fig. 30, middle segment 382 traverses a laparscopic cannula, trocar sleeve, or instrument holder as decribed herein, while proximal end segments 384, 386, and 388 are located outside the patient and distal end segments 390, 392, and 394 are located inside the patient. An operative tip 396 is disposed at one end of the shaft 380, more particularly at a free end of distal end segment 394, and actuator handles or hand grips 398 are disposed at an opposite end of the shaft, more particularly at a free end of proximal end segment 388. Actuator handles 398 are operatively connected to operative tip 396 via shaft 380 for controlling the operation of the tip.

Proximal end segments 384, 386 and 388 form a proximal shaft portion 400 that is independently bendable to form, for example, a C shaped configuration. Proximal end segments 384, 386, and 88 are connected to one another via joints or articulations 402 and 404 and to middle segment 382 via a joint or articulation 406.

Distal end segments 390, 392 and 394 form a distal shaft portion 408 that is independently bendable to form, for example, a C shaped configuration. Distal end segments 390, 392, and 394 are connected to one another via joints or articulations 410 and 412 and to middle segment 382 via a joint or articulation 414.

Operative tip 396 may be rotatable about a longitudinal axis 415. Further rotational capability may be provided by including a joint 416, 418, 420 along distal end segments 90, 392 or middle segment 382, where relative rotation of proximal and distal parts is effectuated about a longitudinal axis of the respective segment.

Proximal end portion 388 is provided with rotary actuators or knobs 422 for modifying the angles between adjacent distal end segments 390, 392, 394, for rotating operative tip 396 relative to distal end segment 394 about axis 414, and for implementing the longitudinal-axis rotation at joints 416, 418, and/or 420. Wing-nut-type clamps 424 may be provided at knobs 422 for releasably locking those actuators to maintain the angles between adjacent distal end segments 30, 92, 94, the rotary position of operative tip 96, and the longitudinal-axis rotation at joints 416, 418, and/or 420.

Clamping elements 426, 428, 430 may be provided at the articulations or joints 402, 404, 406 for locking the relative positions of middle segment 382, and proximal end segments 384, 386, 388. Alternatively, further knobs and wing-nut clamps (not shown) may be provided at the proximal end of the instrument for changing the angles between pairs of adjacent segments 382, 384, 386, 388.

During a laparoscopic surgical procedure, the axial position of operative tip 396 may be adjusted by sliding the laparoscopic instrument of Fig. 30 into and out of the patient, for example, by modifying the position of middle segment 382 relative to the respective cannula or instrument holder aperture. In addition, the axial position of operative tip 396 may be changed by adjusting the configuration of distal end portions 390, 392, 394 relative to one another. Strongly arced configurations have a shorter axial extent than configurations with more shallow arcs. Further degrees of freedom in the positioning of operative tip 396 relative to a surgical site are provided by the rotatability of operative tip 396 about axis 414 and the rotatability at joints 416, 418, 420. The positional adjustability provided by articulations or joints 410, 412, 414 greatly enhances the practical capabilities of the instrument.

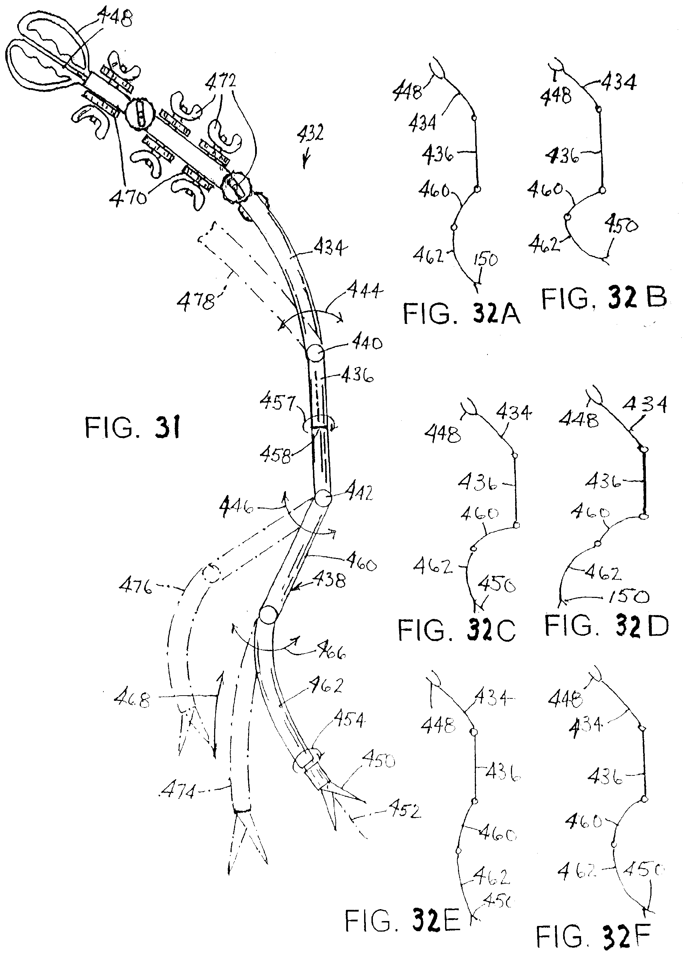

FIG. 31 depicts another stand-alone laparoscopic medical instrument having a shaft 432 insertable through a laparoscopic trocar sleeve or cannula. Shaft 432 has a continuously flexible proximal end portion or segment 434, a rigid straight middle portion or segment 436, and a distal end portion 438. Proximal end portion 434 and distal end portion 436 are connected to opposite ends of middle portion 436 via respective articulations or joints 440 and 442, so that the proximal end portion and the distal end portion are laterally swingable relative to the middle portion, as indicated by dual headed arrows 444 and 446. Middle portion 436 constitutes about one-third of the total length of shaft 432.

Shaft 432 is provided at a proximal end, i.e., at the free end of proximal end portion 434, with a pair of hand grip actuators 448, and is further provided at a distal end, i.e., at the free end of distal end portion 438 with an operative tip 450 such as a scissors, a forceps, a clamp, a cauterizing element, etc. Operative tip 450 is rotatable about a longitudinal axis 452 relative to the end of distal end portion 438, as indicated by a bidirectional arrow 454. As indicated by another bidirectional arrow 457, proximal end portion 434 and distal end portion 438 may be rotable relative to one another about a longitudinal instrument axis 456, owing to a rotable joint 458 exemplarily provided along middle portion 436.

Distal end portion 438 includes two segments or sections 460 and 462 pivotably connected to one another via an articulation or joint 464, as indicated by a dual headed arrow 466. Distal-most section 462 is continuously bendable along its length into an infinite number of smoothly curved generally C-shaped configurations, as indicated by an arrow 468. The more proximal section 460 may be rigid and linear or, alternatively, also continuously flexible along substantially its entire length and formable into a multitude of smoothly arced generally C-shaped configurations.

Proximal end portion 434 is provided along a linear proximal section (not seaprately labeled) with a pluraluty of actuator knobs 470 and locking elements 472 for controllably modifying (a) the degree of curvature of proximal end portion 434 and distal end portion 438, particularly distal-most section 462, (b) the angles between portions 434 and 436 and portions 436 and 438, (c) the angle between sections 460 and 462, (d) the degree and direction of rotation of operative tip 450 about axis 452, and (e) the relative angular position of proximal end portion 434 and distal end portion 438, as determined by the operational status of joint 458. By way of illustration, a modified position and curvature of distal-most section 462 is indicated in Fig. 31 at 474. A modified position of proximal section 460 and a corresponding modified curvature of distal most section 462 are indicated in phantom at 476. An alternate position of proximal end portion 434 with respect to middle portion 436 is shown in phantom at 478.

Figs. 32A-32F depict additional possible positional and curvature configurations of the instrument of Fig. 31, particularly distal end portion 438.

During a laparoscopic surgical procedure, the axial position of operative tip 450 may be adjust by sliding the laparoscopic instrument of Fig. 31 into and out of the patient, for example, by modifying the position of middle portion 436 relative to the respective cannula or instrument holder aperture. In addition, the axial position of operative tip 450 may be changed by adjusting the configuration of distal end portion 438, as depicted in Figs. 32A-32F. Strongly arced configurations (Figs. 32B and 32C) have a shorter axial extent than configurations with more shallow arcs (Figs. 32A, 32E). Further degrees of freedom in the positioning of operative tip 440 relative to a surgical site are provided by the rotatability of operative tip 450 about axis 452 and the rotatability at joint 458.

One or more of the actuator mechanisms including knobs 470 and locking elements 472 may be operatively connected to shaft 432 for bending distal section 462 (and optionally section 460) in a direction out of the plane of the drawing sheet.

Where proximal portion 434 of shaft 432 assumes a first C-shaped configuration in response to operation of a respective one of the knobs 470 and distal portion 438 (or 462) of the shaft assumes a second C-shaped configuration in response to operation of a second one of the knobs 470, the C-shaped configurations may face opposite sides of the shaft, thus forming shaft 432 into a generally S-shape.

As depicted in Fig. 33, a holder 480 for cannulas and laparoscopic surgical instruments such as those discussed above with reference to Figs. 30-32F includes a plate member 482 having a surrounding or perimetric edge 484 and a wall 486 surrounding the plate member. Wall 486 is provided with a plurality of anchoring elements such as eyelets 487 or hooks 489 for securing the holder 480 to a patient via suture thread.

Wall 486 is connected to plate member 482 all along edge 484. Wall has a longitudinal axis 488, with plate member 482 extending substantially transversely to that axis. Plate member 482 is provided with a plurality of apertures or port members 490, 492 for receiving respective elongate laparoscopic instruments 494 and 496. Instrument 494 is configurable to have an S-shaped shaft 498 and may specifically take the form of the instruments discussed with reference to Figs 30 and 31-32F. Instrument 496 is a fiberoptic instrument including a camera 500 in the form of a charge coupled device and a bendable shaft 502. Shaft 502 has a proximal end portion 560 and a distal end portion 562 that may be independently flexed into continuous smooth C-shaped configurations as shown in the drawing. Alternatively, shaft 502 may be substantially identical to shaft 432 of the instrument shown in Fig. 31. The rotational capability discussed above with reference to operative tip 450 and joint 458 may be omitted from laparoscope 496. Laparoscope 496 has an operative tip 564 provided with the usual illumination aperture and imaging lens (neither shown). Actuators are omitted from the depiction in Fig. 33 of instruments 494 and 496 for purposes of simplicity. An actuator for controlling the operative tip 564 of laparoscope 496 may take the form of conventional controls for illumination and CCD operation, where a CCD is located at the operative tip of the device.

Holder 480 is an inflatable unit, both plate 482 and wall 186 being at least partially hollow for receiving a pressurizing fluid such as air. To that end, a tube 504 is connected to holder 480 for the delivery of air from a pressure source such as a syringe (not illustrated). A valve 506 is provided on tube 504. A second tube 508 with a valve 510 is connected to holder 580 for providing a channel for the conveyance of an insufflation gas such as carbon dioxide from a reservoir thereof (not shown) to the patient. An aperture 512 is provided along an inner surface 514 of wall 486 for enabling the delivery of the insufflation gas to the patient via tube 508.

Wall 486 has a height dimension H1 at least as great as, and preferably substantially greater than, a height dimension H2 of plate member 482. Wall 486 has two end portions 516 and 518 extending as endless or annular flanges to plate member 482. Plate member 482 is located towards an upper end of wall 486, plate member 482 forming a shallow cup shape and a deep cup shape with flanges 516 and 518, respectively.

Wall 486 has inner diameters D1 and D2 of the free ends of flanges 516 and 518, opposite plate member 482. These inner diameters D1 and D2 are larger than a diameter D3 of plate member 482, which is the inner diameter of wall 482 at the plate member. Consequently, cannula and instrument holder 480 has a flared or tapered profile on each side of plate 482. This flared or tapered shape may exhibit a curved or arced profile as shown in the drawing.

Although the invention has been described in terms of particular embodiments and applications, one of ordinary skill in the art, in light of this teaching, can generate additional embodiments and modifications without departing from the scope of the claimed invention. Accordingly, it is to be understood that the drawings and descriptions herein are proffered by way of example to facilitate comprehension of the invention and should not be construed to limit the scope thereof.

## Claims

1. A laparoscopic surgical port assembly (100) comprising:
an annular holder (106) disposable in an opening in a patient;
a cannula unit (102) comprising a base (110) in rotatable engagement with said holder so that said cannula unit when attached is rotatable with respect to said holder about a longitudinal axis of said holder, the base (110) is removably attachable to said holder and comprising a planar panel (112) defining a planar closure surface extending substantially tangentially to the patient's skin at said opening during a surgical procedure; and
a plurality of cannulas (118, 120, 122) connected to said base and defining an access path through said closure surface, said cannulas extending at an acute angle relative to said closure surface so that said cannulas are all inclined relative to the patient's skin surface at least in a relaxed or rest configuration of the port assembly.

2. The port assembly defined in claim 1 wherein said cannula unit is partially insertable into said holder.

3. The port assembly defined in claim 2, further comprising a connector member (104) for removably attaching said cannula unit to said holder, said connector also being partially insertable into said holder.

4. The port assembly defined in claim 3 wherein said holder and said connector member are provided with cooperating locking elements (142, 144, 139, 140).

5. The port assembly defined in claim 4 wherein said locking elements include projections (142, 144) on one of said holder and said connector member and further include slots (138, 140) on the other of said holder and said connector member.

6. The port assembly defined in claim 7 wherein said cannula unit includes a base (110) insertable into said holder and wherein said connector includes a portion (132) insertable into said holder to secure said cannula unit to said holder.

7. The port assembly defined in claim 2 said holder is provided internally with a shoulder (156) engaging a lower end (114) of said cannula unit.

8. The port assembly defined in claim 1 wherein said holder is provided with means for releasably securing said holder to the patient.

9. The port assembly defined in claim 1 wherein said cannula unit includes a plurality of cannula members defining respective access paths for surgical instruments.

10. The port assembly defined in claim 2 wherein the closure surface is located in a main plane and said cannulas have linear configurations, each of said cannulas defining a respective secondary plane perpendicular to said main plane, said base having a longitudinal axis, each said secondary plane being spaced from said longitudinal axis.

11. The port assembly defined in claim 2, wherein said cannula is flexible.

12. The port assembly defined in claim 2 or 11, wherein said cannula is flexible and has a relaxed or unstressed configuration that is linear.

## Patentansprüche

1. Laparoskopische chirurgische Anschlussanordnung (100), umfassend:
eine ringförmige Halterung (106), die in einer Öffnung in einem Patienten angeordnet werden kann;
eine Kanüleneinheit (102), umfassend eine Basis (110), die drehbar in die Halterung eingreift, so dass die Kanüleneinheit, wenn sie befestigt ist, in Bezug auf die Halterung um eine Längsachse der Halterung drehbar ist, wobei die Basis (110) lösbar an der Halterung befestigt werden kann und eine flache Platte (112) umfasst,
die eine flache Verschlussfläche definiert, die sich während eines chirurgischen Verfahrens im Wesentlichen tangential zu der Haut des Patienten an der Öffnung erstreckt; und
eine Vielzahl von Kanülen (118, 120, 122), die mit der Basis verbunden sind und einen Zugangsweg durch die Verschlussfläche definieren, wobei sich die Kanülen in einem spitzen Winkel in Bezug auf die Verschlussfläche erstrecken, so dass die Kanülen alle
in Bezug auf die Oberfläche der Haut des Patienten zumindest in einer entspannten oder Ruhekonfiguration der Anschlussanordnung geneigt sind.

2. Anschlussanordnung nach Anspruch 1, wobei die Kanüleneinheit teilweise in die Halterung einführbar ist.

3. Anschlussanordnung nach Anspruch 2, weiter ein Verbindungselement (104) umfassend, um die Kanüleneinheit lösbar an der Halterung zu befestigen, wobei die Verbindung ebenso teilweise in die Halterung einführbar ist.

4. Anschlussanordnung nach Anspruch 3, wobei die Halterung und das Verbindungselement mit zusammenwirkenden Verriegelungselementen (142, 144, 139, 140) bereitgestellt sind.

5. Anschlussanordnung nach Anspruch 4, wobei die Verriegelungselemente Erhebungen (142, 144) auf dem einem der Halterung und des Verbindungselements einschließen und weiter Schlitze (138, 140) auf dem anderen der Halterung und des Verbindungselements einschließen.

6. Anschlussanordnung nach Anspruch 7, wobei die Kanüleneinheit eine Basis (110) einschließt, die in die Halterung einführbar ist, und wobei die Verbindung einen Abschnitt (132) einschließt, der in die Halterung einführbar ist, um die Kanüleneinheit an der Halterung zu fixieren.

7. Anschlussanordnung nach Anspruch 2, wobei die Halterung innen mit einer Schulter (156) bereitgestellt ist, die in ein unteres Ende (114) der Kanüleneinheit eingreift.

8. Anschlussanordnung nach Anspruch 1, wobei die Halterung mit Mitteln zur lösbaren Fixierung der Halterung an dem Patienten bereitgestellt ist.

9. Anschlussanordnung nach Anspruch 1, wobei die Kanüleneinheit eine Vielzahl von Kanülenelementen einschließt, die jeweils Zugangswege für chirurgische Instrumente definieren.

10. Anschlussanordnung nach Anspruch 2, wobei die Verschlussfläche sich in einer Hauptebene befindet und die Kanülen lineare Konfigurationen aufweisen, wobei jede der Kanülen jeweils eine sekundäre Ebene definiert, die senkrecht zu der Hauptebene liegt, wobei die Basis eine Längsachse aufweist, wobei jede der sekundären Ebenen in einem Abstand zu der Längsachse angeordnet ist.

11. Anschlussanordnung nach Anspruch 2, wobei die Kanüle elastisch ist.

12. Anschlussanordnung nach Anspruch 2 oder 11, wobei die Kanüle elastisch ist und eine entspannte oder unbelastete Konfiguration aufweist, die linear ist.

## Revendications

1. Ensemble port chirurgical laparoscopique (100) comprenant :
un support annulaire (106) pouvant être disposé dans une ouverture chez un patient ;
une unité à canules (102) comprenant une base (110) en prise rotative avec ledit support de telle sorte que ladite unité à canules quand elle est attachée peut tourner par rapport audit support autour d'un axe longitudinal dudit support, la base (110) peut être attachée de manière amovible audit support et comprenant un panneau planaire (112) définissant une surface de fermeture planaire s'étendant de manière sensiblement tangentielle à la peau du patient au niveau de ladite ouverture durant une procédure chirurgicale ; et
une pluralité de canules (118, 120, 122) reliées à ladite base et définissant un chemin d'accès à travers ladite surface de fermeture, lesdites canules s'étendant selon un angle aigu par rapport à ladite surface de fermeture de telle sorte que lesdites canules sont toutes inclinées par rapport à la surface de peau du patient au moins dans une configuration relâchée ou de repos de l'ensemble port.

2. Ensemble port selon la revendication 1 dans lequel ladite unité à canules peut être partiellement insérée dans ledit support.

3. Ensemble port selon la revendication 2, comprenant en outre un élément raccord (104) pour attacher de manière amovible ladite unité à canules audit support, ledit raccord pouvant également être partiellement inséré dans ledit support.

4. Ensemble port selon la revendication 3 dans lequel ledit support et ledit élément raccord sont pourvus d'éléments de verrouillage coopérants (142, 144, 139, 140).

5. Ensemble port selon la revendication 4 dans lequel lesdits éléments de verrouillage incluent des projections (142, 144) sur l'un parmi ledit support et ledit élément raccord et incluent en outre des fentes (138, 140) sur l'autre parmi ledit support et ledit élément raccord.

6. Ensemble port selon la revendication 7 dans lequel ladite unité à canules inclut une base (110) pouvant être insérée dans ledit support et dans lequel ledit raccord inclut une portion (132) pouvant être insérée dans ledit support pour fixer ladite unité à canules audit support.

7. Ensemble port selon la revendication 2 dans lequel ledit support est intérieurement pourvu d'un épaulement (156) entrant en prise avec une extrémité inférieure (114) de ladite unité à canules.

8. Ensemble port selon la revendication 1 dans lequel ledit support est pourvu d'un moyen pour fixer de manière détachable ledit support au patient.

9. Ensemble port selon la revendication 1 dans lequel ladite unité à canules inclut une pluralité d'éléments canules définissant des chemins d'accès respectifs pour instruments chirurgicaux.

10. Ensemble port selon la revendication 2 dans lequel la surface de fermeture est située dans un plan principal et lesdites canules ont des configurations linéaires, chacune desdites canules définissant un plan secondaire respectif perpendiculaire audit plan principal, ladite base ayant un axe longitudinal, chaque dit plan secondaire étant espacé dudit axe longitudinal.

11. Ensemble port selon la revendication 2, dans lequel ladite canule est flexible.

12. Ensemble port selon la revendication 2 ou 11, dans lequel ladite canule est flexible et a une configuration relâchée ou non contrainte qui est linéaire.
